# EUROPÄISCHE PATENTSCHRIFT

(11) **EP 0 407 681 B1**
(45) Veröffentlichungstag und Bekanntmachung des Hinweises auf die Patenterteilung: **15.09.1993**
(21) Anmeldenummer: 89890187.1
(22) Anmeldetag: 13.07.1989
(51) Int. Cl.: A61K 6/00, A61K 6/097, A61C 13/23

(54) **Hafteinlage für Zahnprothesen und Verfahren zu deren Herstellung**
Adhesive insert for dentures, and method for its production
Insert adhésif pour prothèses dentaires et son procédé de préparation

(43) Veröffentlichungstag der Anmeldung: 16.01.1991
(73) Patentinhaber: Altwirth, Oskar, A-4950 Altheim (AT)
(72) Erfinder: Altwirth, Oskar, A-4950 Altheim (AT)
(74) Vertreter: Hübscher, Helmut, Dipl.-Ing.

(56) Entgegenhaltungen:
- EP-A- 0 353 375
- DE-A- 2 413 380
- DE-A- 3 546 367
- DE-B- 1 566 252
- WPIL, FILE SUPPLIER, AN 82-62652E(30), Derwent Publications Ltd, London, GB; & JP-A-57 099 511 (LION CORP.)

## Beschreibung

Die Erfindung bezieht sich auf eine Hafteinlage für Zahnprothesen, bestehend aus einem haftstoffdurchsetzten Faservlies sowie auf ein Verfahren zum Herstellen solcher Hafteinlagen.

Um die Haftfähigkeit und Wirkungsdauer rein pastöser Haftmittel für Zahnprothesen zu erhöhen, gibt es gemäß der DE-C-24 13 380 bereits Hafteinlagen aus einem Faservlies, das mit Haftstoff durchsetzt ist, welches Faservlies beim Einsatz der Hafteinlage zwischen Prothese und Kiefer durch seine Fasern gewissermaßen eine Armierung des Haftstoffes bildet und das Ausspülen des Haftstoffes verzögern soll. Allerdings ist bei den bekannten Hafteinlagen das Faservlies zusammen mit einem trockenen, pulverförmigen Hydrocolloid als Haftstoff verpreßt, das durch ein Befeuchten aufquellt und aktiviert wird, so daß es eine gewisse Menge Flüssigkeit bis zur Sättigung aufnehmen und dadurch einen Adhäsionseffekt bewirken, doch auch durch Flüssigkeit und Speichel ausgespült werden kann. Die Wirkungsdauer dieser Hydrocolloide oder andere Quellmittel enthaltenden Hafteinlagen bleibt daher unbefriedigend; sie sind nicht in der Lage, insbesondere Unterkiefer-Zahnprothesen wegen ihrer recht geringen Haftflächen mit gewünschter Sicherheit und Wirkungsdauer zu fixieren.

Der Erfindung liegt daher die Aufgabe zugrunde, diese Mängel zu beseitigen und eine Hafteinlage der eingangs geschilderten Art zu schaffen, die sich durch ihre besonders gute und lang anhaltende Haftwirkung auszeichnet. Außerdem soll ein Verfahren zur rationellen Herstellung solcher Hafteinlagen angegeben werden.

Die Erfindung löst diese Aufgabe dadurch, daß das Faservlies einen Haftstoff aus einer honigähnlichen, zähflüssigen Mischung aufnimmt, die sich aus Polyvinylacetat und einem physiologisch einwandfreien, wasserunlöslichen Lösungsmittel, wie Glycerinriacetat oder Propylenglykol, sowie wenigstens einem Hydrocolloid, wie Alginat und/oder Carboxymethylcellylose, zusammensetzt. Dieser Haftstoff erlaubt nicht nur die Nutzung seiner Vorzüge als Kleber, wie Abspülsicherheit durch die Einbettung des Alginates bzw. der Carboxymethylcellulose in Polyvinylacetat und der durch das Polyvinylacetat selbst gegebene Klebeeffekt für die Hafteinlage, sondern bringt darüber hinaus noch einen besonders wirkungsvollen Unterdruck-Saugeffekt mit sich. Wird nämlich die Einlage zwischen Prothese und Kiefer eingesetzt, entsteht durch Feuchtigkeitsaufnahme aus dem Haftstoff eine kompakte, gummiartige Masse, die durch das Faservlies eine strukturelle Festigung erfährt. Diese an den Haftflächen angepreßte Masse ergibt auf Grund der Druck- und Zugbelastung bei Kaubewegungen u. dgl. stark haftende Unterdruck- und Saugbereiche. Dieser Unterdruck-Saugeffekt garantiert zusammen mit dem Klebeeffekt der Einlage eine überraschend dauerhafte und fest haltende Haftverbindung zwischen Prothese und Kiefer und ermöglicht damit ein langzeitiges, sicheres Tragen auch von Unterkiefer-Zahnprothesen. Durch die Verwendung eines wasserunlöslichen Lösungsmittels wird dabei im Gegensatz zu anderen ähnlichen Haftmitteln (DE-C-35 46 367; WPIL, FILE SUPPLIER, AN 82-62652 E (30) und JP-A-57099511 oder die nicht vorveröffentlichte EU-Anmeldung 88 89 0202.0) auf Alkohol völlig verzichtet und eine optimale Ausschwemmsicherheit erreicht, da Alkohol als Lösungsmittel natürlich auch die Haftstoffmasse an sich wasserlöslich macht. Ein entsprechendes wasserunlösliches Lösungsmittel, das selbstverständlich physiologisch unbedenklich sein muß, läßt sich nicht ausschwemmen und gewährleistet daher die gewünschte Haltbarkeit. Außerdem verhindert es ein Austrocknen der Hafteinlage auch bei schlechter Verpackung.

Sind auf das mit dem Haftstoff imprägnierte Faservlies beidseits Deckfolien aufgelegt, ergibt sich ein hygienischer Schutz und eine saubere Handhabung.

Gemäß einer vorteilhaften Weiterbildung der Erfindung wird zur rationellen Herstellung einer wirkungsvollen Hafteinlage eine Lösung aus Polyvinylacetat und einem physiologisch einwandfreien Lösungsmittel, wie Glycerintriacetat oder Propylenglykol, vorbereitet, werden in diese Lösung Alginat und/oder Carboxymethylcellulose oder andere Hydrocolloide eingebracht und wird die ganze Mischung auf ca. 70°C erwärmt und zu einer zähflüssigen, honigartigen Masse verrührt, mit welcher Masse dann das Faservlies durchsetzt bzw. imprägniert wird. Dabei wird günstigerweise eine Lösung aus etwa 70% Polyvinylacetat und 30% Lösungsmittel aufbereitet und etwa 100 Gewichtsteile dieser Lösung werden mit 74 Gewichtsteilen Alginat oder Carboxymethylcellulose bzw. 34 Gewichtsteilen Alginat und 40 Gewichtsteilen Carboxymethylcellulose vermischt. Eine Mindesttemperatur von 70°C ist dabei notwendig, um das Polyvinylacetat in Lösung zu bringen und in diese Lösung kommen dann bei gleicher Temperatur Alignat und/oder Carboxymethylcellulose, worauf die ganze Mischung in einem üblichen Rührwerk bis zu einer zähflüssigen, honigähnlichen Masse verrührt wird. In einem eigenen Behälter wird sodann das Faservlies ebenfalls bei 70°C mit der Masse imprägniert, das anschließend durch ein Abstreifsystem durchgezogen wird. Es entsteht ein Haftstoff, der im heißen Zustand zähflüssig ist, im abgekühlten Zustand im Faservlies eine feste, kompakte, vom Wasser kaum auflösbare, stark klebende Substanz ergibt und dabei die gewünschte Klebe-Saugwirkung erbringt.

Besonders günstig ist es weiters, wenn erfindungsgemäß das Faservlies mit heißem Haftstoff imprägniert und dann dünn abgestreift wird, worauf das imprägnierte Faservlies beidseitig mit Deckfolien versehen wird, aus welchem so entstehenden Band anschließend der Prothesenform entsprechende Stanzlinge ausgestanzt und diese als gebrauchsfertige Hafteinlagen abgepackt werden. Zur Herstellung der Hafteinlagen wird eine endlos zugeführte Faservliesbahn durch einen Behälter mit den auf 70°C erwärmten Haftstoff gezogen. Das Faservlies nimmt den zähflüssigen, heißen Haftstoff auf und wird dann weiter durch ein Abstreifsystem geführt, um die überflüssige Masse abzustreifen und eine gewisse Stärke zu erreichen. Das so imprägnierte Faservlies kann nun in einer Rollvorrichtung beidseitig durch Deckfolien abgedeckt werden, bevor es durch ein Walzensystem auf die notwendige Stärke von 0,5 mm gebracht wird. Das vorgefertigte Band gelangt dann in eine Prägestanzeinrichtung, in der entsprechende Stanzlinge ausgestanzt werden. Auf wirtschaftliche Weise entstehen fertig abgepackte, jederzeit einsetzbare Hafteinlagen, die vor einem Austrocknen geschützt und daher lang lagerfähig bleiben. Es sind dünne Hafteinlagen, die beiderseits gleiche Klebewirkung haben und elastisch genug sind, um leicht auf die Zahnprothese aufgebracht zu werden.

Die erfindungsgemäße Hafteinlage zeichnet sich dadurch aus, daß die fertige Klebemasse in heißem Zustand in ein einlagiges Faservlies eingebracht wird und dann in erhärtetem Zustand eine dünne Einlage ergibt. Auf Grund der Dünnschichtigkeit des Einfachvlieses besteht keine Gefahr einer Bißerhöhung, da die Durchschnittsstärke des Einfachvlieses nur bis zu 0,5 mm beträgt, während übliche Einlagen eine Stärke von 1,5 mm besitzen.

Bei der erfindungsgemäßen Hafteinlage kann nur eine kleine Menge Flüssigkeit, bedingt durch die fest eingebetteten Hydrocolloide, in das Faservlies eindringen, doch ist diese leichte Aufschwemmung notwendig, um den Saugeffekt zu erreichen. Daß der Saugeffekt langanhaltend wirksam ist, erklärt sich durch das nur spärliche Aufnehmen von Flüssigkeiten, die dann den Saugeffekt bewirken, wobei zwar das notwendige Eindringen von etwas Flüssigkeit gewährleistet, aber das Ausschwemmen des Haftstoffes aus dem Faservlies verhindert ist.

Da das Faservlies nicht mit einem aus Alkohol als Lösungsmittel entstandenen Haftmittel versorgt wird, das natürlich keine Verbindung mit dem Faservlies eingehen kann und daher ausgeschwemmt wird, sondern bei großer Wärme mit einer zähflüssigen Substanz, die durch das Erstarren fest und kompakt wird und sich mit dem Vlies verbindet, imprägniert wird, ist ein besonders starker und lang dauernder Klebe- und Saugeffekt garantiert.

## Patentansprüche

1. Hafteinlage für Zahnprothesen, bestehend aus einem haftstoffdurchsetzten Faservlies, dadurch gekennzeichnet, daß das Faservlies einen Haftstoff aus einer honigähnlich, zähflüssigen Mischung aufnimmt, die sich aus Polyvinylacetat und einem physiologisch einwandfreien, wasserunlöslichen Lösungsmittel, wie Glycerintriacetat oder Propylenglykol, sowie wenigstens einem Hydrocolloid, wie Alginat, und/oder Carboxymethylcellylose, zusammensetzt.

2. Hafteinlage nach Anspruch 1, dadurch gekennzeichnet, daß auf das mit dem Haftstoff imprägnierte Faservlies beidseits Deckfolien aufgelegt sind.

3. Verfahren zum Herstellen einer Hafteinlage nach Anspruch 1 oder 2, dadurch gekennzeichnet, daß eine Lösung aus Polyvinylacetat und einem physiologisch einwandfreien, wasserunlöslichen Lösungsmittel, wie z.B. Glycerintriacetat oder Propylenglykol, vorbereitet, in diese Lösung Alginat und/oder Carboxymethylcellulose oder andere Hydrocolloide eingebracht werden und die ganze Mischung auf ca. 70°C erwärmt und zu einer zähflüssigen, honigartigen Masse verrührt wird, mit welcher Masse dann das Faservlies durchsetzt bzw. imprägniert wird.

4. Verfahren nach Anspruch 3. dadurch gekennzeichnet, daß eine Lösung aus etwa 70% Polyvinylacetat und 30% Glycerintriacetat bzw. Propylenglykol aufbereitet wird und etwa 100 Gewichtsteile dieser Lösung mit 74 Gewichtsteilen Alginat oder Carboxymethylcellulose bzw. 34 Gewichtsteilen Alginat und 40 Gewichtsteilen Carboxymethylcellulose vermischt werden.

5. Verfahren nach Anspruch 3 oder 4, dadurch gekennzeichnet, daß das Faservlies mit heißem Haftstoff durchsetzt bzw. imprägniert und dann dünn abgestreift wird, worauf das imprägnierte Faservlies beidseitig mit Deckfolien versehen wird, aus welchem so entstehenden Band anschließend der Prothesenform entsprechende Stanzlinge ausgestanzt und diese als gebrauchsfertige Hafteinlagen abgepackt werden.

## Claims

1. An adhesive insert for dental prostheses comprising a non-woven fabric permeated by adhesive, characterised in that the non-woven absorbs an adhesive in the form of a honey-like viscous mixture consisting of polyvinyl acetate and a physiologically acceptable water-insoluble solvent such as glycerol triacetate or propylene glycol, and at least one hydrocolloid such as alginate and/or carboxymethyl cellulose.

2. An adhesive insert according to claim 1, characterised in that cover sheets are disposed on both sides of the non-woven fabric impregnated with the adhesive.

3. A method of producing an adhesive insert according to claim 1 or 2, characterised in that a solution of polyvinyl acetate and a physiologically acceptable water-soluble solvent such as glycerol triacetate or propylene glycol is prepared, alginate and/or carboxymethyl cellulose or other hydrocolloids are incorporated in the solution, and the entire mixture is heated to about 70°C and agitated to form a viscous honey-like material, which is then used to permeate or impregnate the non-woven fabric.

4. A method according to claim 3, characterised in that a solution of about 70% polyvinyl acetate and 30% glycerol triacetate or propylene glycol is prepared and about 100 parts by weight of the solution is mixed with 74 parts by weight of alginate or carboxymethyl cellulose or 34 parts by weight of alginate and 40 parts by weight of carboxymethyl cellulose.

5. A method according to claim 3 or 4, characterised in that the non-woven fabric is permeated or impregnated with hot adhesive and then scraped thin, after which the impregnated non-woven fabric is provided with cover sheets on both sides to form a strip from which blanks corresponding to the shape of the prosthesis are punched out and packed as adhesive inserts ready for use.

## Revendications

1. Garniture adhésive pour prothèse dentaire, composée d'un matelas de fibres, imprégné d'un adhésif,
caractérisée en ce que le matelas en fibres reçoit un adhésif composé d'un mélange mielleux, liquide visqueux, composé d'acétate de polyvinyle et d'un solvant physiologiquement parfait, insoluble dans l'eau, tel que du triacétate de glycérol ou de polypropylène-glycol, ainsi que d'au moins un hydrocolloïde tel que l'alginate et ou la carboxy-méthyl-cellulose.

2. Garniture adhésive selon la revendication 1
caractérisée en ce que des feuilles de couverture sont posées de deux cotés, sur le matelas en fibres imprégné de l'adhésif.

3. Procédé de fabrication d'une garniture adhésive selon la revendication 1 ou 2,
caractérisé en ce que l'on prépare une solution composée d'acétate de polyvinyle et d'un solvant physiologiquement parfait, insoluble dans l'eau, tel que par exemple le triacétate de glycérol ou le polypropylène-glycol, que l'on introduit dans cette solution de l'alginate et/ou de la carboxy-méthyl-cellulose ou d'autre hydro-colloides, et que l'on chauffe la totalité du mélange à 70° C environ, puis que l'on agite pour obtenir une masse liquide visqueuse mielleuse, avec laquelle on traverse, respectivement imprègne ensuite le matelas en fibres.

4. Procédé selon la revendication 3,
caractérisé en ce qu'on prépare une solution composée d'à peu prés 70 % d'acétate de polyvinyle et de 30 % de triacétate de glycérol respectivement de polypropylène-glycol, et que l'on mélange environ 100 parties en poids de cette solution à 74 parties en poids d'alginate ou de carboxy-méthyl-cellulose, respectivement 34 parties en poids d'alginate et 40 parties en poids de carboxy-methyl-cellulose.

5. Procédé selon la revendication 3 ou 4,
caractérisé en ce que le matelas en fibres est traversé, respectivement imprégné avec un adhésif chaud, et ensuite soumis à un raclage, à la suite de quoi le matelas en fibres imprégné est pourvu de deux cotés de feuilles de couverture, d'où l'on opère ensuite, le ruban ainsi obtenu étant ensuite soumis à un estampage d'ébauche correspondant à la forme de la prothèse, puis que l'on emballe cette ébauche, sous forme de garnitures adhésives prêtes à l'emploi.
